(19) Europäisches Patentamt
European Patent Office
Office européen des brevets

(11) **EP 0 839 514 B1**

(12) **EUROPÄISCHE PATENTSCHRIFT**

(45) Veröffentlichungstag und Bekanntmachung des Hinweises auf die Patenterteilung:
**29.05.2002  Patentblatt 2002/22**

(51) Int Cl.7: **A61K 7/06**

(21) Anmeldenummer: **97116838.0**

(22) Anmeldetag: **27.09.1997**

(54) **Haarbehandlungsmittel mit langanhaltenden Festigungseigenschaften**

Hair treating composition with long-lasting fixing properties

Produit pour le traitement des cheveux à pouvoir fixant de longue durée

(84) Benannte Vertragsstaaten:
**DE ES FR GB IT**

(30) Priorität: **16.10.1996  DE 19642622**

(43) Veröffentlichungstag der Anmeldung:
**06.05.1998  Patentblatt 1998/19**

(73) Patentinhaber: **Wella Aktiengesellschaft**
**64274 Darmstadt (DE)**

(72) Erfinder:
• **Schmenger, Jürgen**
**64331 Weiterstadt (DE)**
• **Karlen, Thomas, Dr.**
**3013 Bern (CH)**
• **Steinbrecht, Karin, Dr.**
**64372 Ober-Ramstadt (DE)**
• **Borth, Silvia**
**64407 Fränkisch-Crumbach (DE)**

(56) Entgegenhaltungen:
**WO-A-93/23009        WO-A-95/03776**

**Beschreibung**

Gegenstand der vorliegenden Erfindung ist ein Haarbehandlungsmittel mit einem Gehalt an mindestens einem nichtionischen, wasserunlöslichen Vinyl/Silikon Copolymer mit einem Grundgerüst aus polymeren Siloxaneinheiten und Seitenketten aus polymeren Vinyleinheiten sowie mit einem Gehalt an mindestens einem schwerflüchtigen Silikonderivat.

Haarbehandlungsmittel mit einer haarfestigenden Wirkung sind hinreichend bekannt. Die für diese Zwecke üblicherweise eingesetzten kosmetischen Polymere zeigen in wäßrigen oder alkoholischen Lösungen gute Festigungseigenschaften, die nach der Anwendung mehr oder weniger gut die Haare verformen und festigen, die jedoch spätestens nach dem Shamponieren wieder herausgewaschen werden. Eine dauerhafte Verformung der Haare, über mehrere Haarwäschen hinweg, ist bisher nur über einen chemischen Eingriff in die Haarstruktur mittels einer Dauerwellbehandlung zu erzielen.

In der WO 93/23009 und der WO 95/03776 werden kosmetische Mittel mit einem Gehalt an Vinyl/Silikon Copolymeren mit einem Silikongrundgerüst und Acrylat-Seitenketten beschrieben. Handelt es sich bei diesen Polymeren um nichtionische, hydrophobe Substanzen, so werden sie in Lippenstiften, Lidschattenstiften oder Antitranspirantsprays zur Erzielung von pflegenden Wirkungen eingesetzt. Handelt es sich bei den beschriebenen Vinyl/Silikon Copolymeren um ionische, hydrophilere Substanzen, so können sie auch in Haarsprays zur Erzielung einer haarfestigenden Wirkung eingesetzt werden.

Aufgrund der Hydrophilie der ionischen Copolymere ist jedoch keine langanhaltende, über mehrere Haarwäschen hinweg wirksame Festigung zu erzielen.

Es bestand daher die Aufgabe, ein Haarbehandlungsmittel zur Verfügung zu stellen, welches die große Lücke zwischen einer Dauerwellbehandlung und der Behandlung mit üblichen Haarfestigern oder Haarsprays ausfüllt. Hierbei sollte sich das Haar trotz der bleibenden Festigung nicht starr und klebrig, sondern geschmeidig und natürlich anfühlen, ohne daß sichtbare Rückstände zu erkennen sind und ohne daß das Haar durch einen chemischen Eingriff in die Haarstruktur geschädigt wird.

Es wurde nun gefunden, daß die gestellte Aufgabe gelöst wird durch ein Haarbehandlungsmittel mit einem Gehalt an

(a) mindestens einem nichtionischen, wasserunlöslichen Vinyl/Silikon Copolymer mit einem Grundgerüst aus polymeren Siloxaneinheiten und Seitenketten aus polymeren Vinyleinheiten und

(b) mindestens einem schwerflüchtigen Silikonderivat.

Das Vinyl/Silikon Copolymer ist in dem erfindungsgemäßen Mittel vorzugsweise in einer Menge von 0,1 bis 50 Gewichtsprozent und das schwerflüchtige Silikonderivat vorzugsweise in einer Menge von 0,01 bis 50 Gewichtsprozent enthalten.

Geeignete Vinyl/Silikon Copolymere sind beispielsweise solche der allgemeinen Formel (I)

$$R^1R^2R^3Si\text{-}[O\text{-}SiR^4R^5\text{-}]_nO\text{-}SiR^6R^7R^8 \tag{I},$$

wobei die Reste $R^1$ bis $R^8$ gleich oder verschieden sein können und unabhängig voneinander für eine Alkyl-, Aryl-, Alkylaryl-, Alkoxy-, Alkylamino-, Fluoroalkyl-, Wasserstoff-, Hydroxy- oder für eine ZSA-Gruppe stehen, Z für eine divalente Verbindungsgruppe, S für Schwefel und A für ein aus nichtionischen Monomeren aufgebautes Vinylpolymer-Segment steht und n eine Zahl größer oder gleich 5 bedeutet mit der Maßgabe, daß mindestens einer der Reste $R^1$ bis $R^8$ für eine ZSA-Gruppe steht.

Geeignete nichtionische Monomere des Vinylpolymer-Segments A sind vorzugsweise ausgewählt aus niedrigpolaren Estern der Acrylsäure oder der Methacrylsäure mit Alkoholen kurzer bis mittlerer Kettenlänge, die das Polymer im wesentlichen wasserunlöslich machen. Geeignete Alkohole sind beispielsweise Ethanol, 1-Propanol, 2-Propanol, 1-Butanol, 1,1-Dimethylethanol, 2-Methyl-1-propanol, 1-Pentanol, 2-Pentanol, 3-Pentanol, 2-Methyl-1-butanol, 3-Methyl-1-butanol, 2-Hexanol, 3-Methyl-1-pentanol, 3-Methyl-1-pentanol, Cyclohexanol, 2-Ethyl-1-butanol, 3-Heptanol, Benzylalkohol, 2-Octanol, 6-Methyl-1-heptanol, 2-Ethyl-1-hexanol, 3,5-Dimethyl-1-hexanol, 3,5,5-Trimethyl-1-hexanol, 1-Decanol, 1-Dodecanol und Ähnliche. Vorzugsweise enthalten die Alkohole 1 bis 18 Kohlenstoffatome, besonders bevorzugt sind Alkohole mit 1 bis 12 Kohlenstoffatomen.

Kleine Mengen an copolymerisierbaren Monomeren wie Styrol, Vinylester, Vinylchlorid, Vinylidenchlorid, Acrylonitril, Methacrylonitril, Acryloxypropyltrimethoxysilan, Methacryloxypropyltrimethoxysilan, andere Acryloyl-Monomere und strukturell ähnliche Monomere können verwendet werden.

Beispiele besonders bevorzugter Monomere beinhalten Isooctylmethacrylat, Isononylmethacrylat, 2-Ethylhexylmethacrylat, Laurylmethacrylat, i-Pentylmethacrylat, n-Butylmethacrylat, i-Butylmethacrylat, Methylmethacrylat, Ethylmethacrylat, t-Butylmethacrylat, Tridecylmethacrylat, Stearylmethacrylat und strukturell ähnliche Monomere sowie Gemische der oben definierten Monomere.

Hochpolare, nichtionogene Monomere können zu einem kleinen Teil im Vinylpolymer-Segment A copolymerisiert sein, wenn dabei das gemäß Anspruch (A) definierte Polymer im wesentlichen wasserunlöslich bleibt. Beispiele hochpolarer, nichtionogener Monomere beinhalten nichtionogene Acrylate oder Methacrylate mit mindestens einer hochpolarisierbaren Gruppe wie einer Hydroxyl-, einer Alkoxy-, einer Aminogruppe (primär, sekundär oder tertiär) oder eines Alkenyl-Heterozyklus, Vinylpyrrolidon oder Monomere mit einer Ethylenoxid oder Propylenoxidseitenkette. Das Vinyl/Silikon Copolymer ist jedoch vorzugsweise frei von Gruppen, welche durch Copolymerisation von hochpolaren, nichtionogenen Monomeren in das Vinylpolymer-Segment A herrühren.

Das Vinylpolymersegment A des Vinyl/Silikon Copolymers ist vorzgsweise frei von anionischen, kationischen oder amphoteren Gruppen, welche durch Einpolymerisieren von anionischen, kationischen oder amphoteren Monomeren herrühren.

Die Herstellung von erfindungsgemäß einsetzbaren Vinyl/Silikon Copolymeren ist in der WO 93/23009 und in der Wo 95/03776 beschrieben.

Besonders bevorzugt als Polymere für das erfindungsgemäße Haarbehandlungsmittel sind Dimethylsiloxan/Methyl-3-mercaptopropyl-siloxan/Isobutylmethacrylat-Copolymere.

Ein geeignetes Produkt wird beispielsweise unter der Bezeichnung Silicone "Plus" Polymer VS 70 von der Firma 3M, St. Paul, Minnesota/USA vertrieben.

Schwerflüchtige Silikonderivate im Sinne der vorliegenden Erfindung sind Silikonderivate mit einer Verdunstungszahl größer als 20. Besonders bevorzugt sind Silikonderivate mit einer Verdunstungszahl von größer 100. Die Verdunstungszahl ist definiert als der Quotient aus der Verdunstungszeit der zu prüfenden Flüssigkeit und der Verdunstungszeit von Diethylether als Vergleichsflüssigkeit.

Die schwerflüchtigen Silikonderivate weisen vorzugsweise eine Viskosität von maximal 100.000 mPa.s, besonders bevorzugt von maximal 20.000 mPa.s bei 25°C auf. Geeignete schwerflüchtige Silikonderivate sind beispielsweise hochmolekulare Polydimethylsiloxane (Dimethicone), Polydimethylsiloxane mit Hydroxylendgruppen (Dimethiconole) oder Dimethylsiloxanglykolcopolymere (Dimethicon Copolyole). Schwerflüchtige Dimethylsiloxanglykolcopolymere werden zum Beispiel unter dem Handelsnamen Abil®-Polyether-Polysiloxan der Firma Goldschmidt/Deutschland, Silwet®-L der Firma Union Carbide/USA oder Silikonfluids 190, 193, Q2-5520 der Firma Dow Corning/USA vertrieben.

Geeignete schwerflüchtige Silikonderivate sind auch cyklische Siloxanpolymere mit einer Verdunstungszahl größer 100 wie sie zum Beispiel unter den Handelsbezeichnungen Dow Corning 244 oder 245 Fluid der Firma Dow Corning/USA, Abil® K4 der Firma Goldschmidt/Deutschland oder GE Silicone 1173 der Firma GE Silicones/USA vertrieben werden.

Besonders bevorzugte schwerflüchtige Silikonderivate sind die Polydimethylsiloxane mit Hydroxylendgruppen (Dimethiconole). Geeignete Dimethiconole werden zum Beispiel unter dem Handelsnamen Silicone Fluid F 212 oder Siliconöl CT 601 M der Firma Wacker/Deutschland, Silicone Fluid Serie NM 201-50000 der Firma Hüls/Deutschland, die S-Serien der Firma Siltech/USA, und Unisil® SF-R der Firma UPI/USA vertrieben.

Geeignete Gemische mit Cyclomethiconen oder Dimethiconen sind die Handelsprodukte Dow Corning Q2-1401 und Dow Corning Q2-1403 der Firma Dow Corning/USA und Abil® OSW 12 und 13 der Firma Goldschmidt/Deutschland.

In einer bevorzugten Ausführungsform enthält das erfindungsgemäße Haarbehandlungsmittel zusätzlich mindestens ein leichtflüchtiges Silikonderivat. Ein leichtflüchtiges Silikonderivat im Sinne der vorliegenden Erfindung ist ein Silikonderivat mit einer Verdunstungszahl von maximal 20.

Geeignete leicht-flüchtige Silikonderivate sind Siloxanpolymere wie sie beispielsweise unter den Handelsbezeichnungen Dow Corning 200 Fluid, Dow Corning 225 Fluid der Firma Dow Corning/USA, Abil® 5000 der Firma Goldschmidt/Deutschland, oder Belsil® DMC der Firma Wacker/Deutschland vertrieben werden.

In einer bevorzugten Ausführungsform enthält das erfindungsgemäße Haarbehandlungsmittel zusätzlich mindestens ein filmbildendes, haarfestigendes, synthetisches oder natürliches Polymer. Dieses zusätzliche Polymer kann nichtionischen, kationischen, anionischen oder amphoteren Charakter haben und wird bevorzugt in einer Menge von 0,01 bis 50 Gewichtsprozent, besonders bevorzugt in einer Menge von 0,5 bis 50 Gewichtsprozent eingesetzt.

Unter filmbildenden Polymeren werden solche Polymere verstanden, welche allein in 0,1 bis 5%iger wäßriger, alkoholischer oder wäßrig-alkoholischer Lösung angewandt in der Lage sind, auf dem Haar einen Polymerfilm abzuscheiden und auf diese Weise das Haar zu festigen.

Geeignete synthetische nichtionische, filmbildende haarfestigende Polymere sind zum Beispiel Homopolymere des Vinylpyrrolidons, die beispielsweise unter den Handelsbezeichnungen Luviskol® K von der Firma BASF, Ludwigshafen/Deutschland oder PVP-K von der Firma ISP, Wayne, NJ/USA vertrieben werden sowie Homopolymere des N-Vinylformamids, die beispielsweise unter der Handelsbezeichnung PVF von der Firma National Starch/USA vertrieben werden. Weitere geeignete synthetische filmbildende, nichtionische haarfestigende Polymere sind zum Beispiel Copolymerisate aus Vinylpyrrolidon und Vinylacetat, die beispielsweise unter den Handelsbezeichnungen Luviskol® VA von der Firma BASF, Ludwigshafen/Deutschland vertrieben werden; Terpolymere aus Vinylpyrrolidon, Vinylacetat und Vinylpropionat, die beispielsweise unter der Handelsbezeichnung Luviskol® VAP der Firma BASF, Ludwigshafen/Deutschland vertrieben werden; Polyacrylamide, die beispielsweise unter den Handelsbezeichnungen Akypomine® P 191 von der Firma CHEM-Y, Emmerich/Deutschland oder Sepigel® 305 von der Firma Seppic/USA vertrieben werden; Polyvinylalkohole, die beispielsweise unter den Handelsbezeichnungen Elvanol® der Firma Du Pont oder Vinol® 523/540 der Firma Air Products/USA vertrieben werden sowie Polyethylenglykole mit einem Molekulargewicht von 800 bis 20.000 g/mol, die beispielsweise unter den Handelsbezeichnungen Lipoxol® 1000 von der Firma Hüls AG/Deutschland, Pluracol® E 4000 von der Firma BASF/Deutschland oder Upiwax® 20.000 von der Firma UPI vertrieben werden.'

Geeignete synthetische filmbildende, anionische Polymere sind zum Beispiel Crotonsäure-Vinylacetat-Copolymere, die beispielsweise in Form einer 60%igen Lösung von Isopropanol/Wasser unter der Handelsbezeichnung Aristoflex® von der Firma Hoechst/Deutschland vertrieben werden. Weitere geeignete anionische Polymere sind zum Beispiel Terpolymere der Acrylsäure, Ethylacrylat und N-t-Butylacrylamid wie sie unter den Handelsnamen Ultrahold 8 und Ultrahold Strong der Firma BASF, Ludwigshafen/Deutschland vertrieben werden.

Geeignete natürliche filmbildende Polymere mit haarfestigender Wirkung sind zum Beispiel niedermolekulares Chitosan mit einem Molekulargewicht von 30.000 bis 70.000 g/mol, welches beispielsweise von der Firma Kyowa Oil & Fat/Japan vertrieben wird. Des weiteren können verschiedene Saccharidtypen verwendet werden wie zum Beispiel Polysaccharide oder Gemische aus Oligo-, Mono- und Disacchariden, welche beispielsweise unter dem Handelsnamen C-PUR® von der Firma Cerestar, Brüssel/Belgien vertrieben werden. Weitere geeignete, natürliche Polymere sind chinesisches Balsamharz und Cellulosederivate, zum Beispiel Hydroxypropylcellulose mit einem Molekulargewicht von 30.000 bis 50.000 g/mol, welche beispielsweise unter der Handelsbezeichnung NISSO SL® von der Firma Lehmann & Voss, Hamburg/Deutschland vertrieben wird.

Geeignete amphotere Polymere sind zum Beispiel Copolymere aus Octylacrylamid, t-Butylaminoethylmethacrylate und zwei oder mehr Monomeren, bestehend aus Acrylsäure, Methacrylsäure oder deren Ester, wie sie zum Beispiel unter dem Handelsnahmen Amphomer® 28-4910 oder Amphomer LV-71 der Firma National Starch/USA erhältlich sind.

Von den kationischen Polymeren, die in dem erfindungsgemäßen Mittel enthalten sein können, sind zum Beispiel Polyvinylpyrrolidon/DimethylaminoethylmethacrylatCopolymer, das unter der Handelsbezeichnung Gafquat® 755 N von der Firma Gaf Co., New York/USA vertrieben wird. Weitere kationische Polymere sind beispielsweise das von der Firma BASF AG/Ludwigshafen/Deutschland unter dem Handelsnamen Luviquat HM 550 vertriebene Copolymer aus Polyvinylpyrrolidon und Imidazoliminmethochlorid, das von der Firma Calgon, Pittsburgh/USA unter dem Handelsnamen Merquat® Plus 3300 vertriebene Terpolymer aus Dimethyldiallylammoniumchlorid, Natriumacrylat und Acrylamid, das von der Firma ISP/USA unter dem Handelsnamen Gaffix® VC 713 vertriebene Terpolymer aus Vinylpyrrolidon, Dimethylaminoethylmethacrylat und Vinylcaprolactam, das von der Firma Amerchol, Edison/USA, unter dem Handelsnamen Polymer JR® vertriebene quaternierte Ammoniumsalz der Hydroxyethylcellulose und einem trimethylammonium-substituierten Epoxid, das von der Firma Gaf unter dem Handelsnamen Gafquat® HS 100 vertriebene Vinylpyrrolidon/Methacrylamidopropyltrimethylammoniumchlorid-Copolymer und das von der Firma Goldschmidt, Essen/Deutschland unter dem Handelsnamen Abil® Quat 3272 vertriebene diquaternäre Polydimethylsiloxan.

Des weiteren können in dem erfindungsgemäßen Mittel Polymere mit verdickender Wirkung eingesetzt werden. Von den Verdickern, die in dem erfindungsgemäßen Mittel enthalten sein können, sind Homopolymere der Acrylsäure mit einem Molekulargewicht von 2.000.000 bis 6.000.000 zu nennen, die beispielsweise von der Firma BF Goodrich, Cleveland/USA unter der Handelsbezeichnung Carbopol® vertrieben werden. Als weitere Verdicker kann das erfindungsgemäße Mittel ein Acrylsäurehomopolymer mit einem Molekulargewicht von 4.000.000 enthalten, das beispielsweise von der Firma BF Goodrich unter der Handelsbezeichnung Carbopol® 940 vertrieben wird. Weitere Verdicker sind beispielsweise die von der Firma BF Goodrich unter dem Handelsnamen Carbopol® ETD 2001 oder von der Firma Protex/Frankeich unter dem Handelsnamen Modarez V 600 PX vertriebene Acrylsäurehomopolymer, das von der Firma Hoechst/Deutschland unter dem Handelsnamen Hostacerin® PN 73 vertriebene Polymer aus Acrylsäure und Acrylamid (Natriumsalz) mit einem Molekulargewicht von 2.000.000 bis 6.000.000 und das von der Firma Alban Muller, Montreuil/Frankreich unter dem Handelsnamen Amigel® vertriebene Sclerotium Gum. Besonders bevorzugt sind die Copolymere der Acrylsäure oder der Methacrylsäure, wie sie zum Beispiel unter dem Handelsnamen Carbopol 1342 oder Pemulen TR1 der Firma Goodrich/USA vertrieben werden.

Das erfindungsgemäße Mittel wird bevorzugt in einem alkoholischen oder in einem wäßrig-alkoholischen Milieu konfektioniert, aber es sind auch wasserfreie Rezepturen möglich. Des weiteren können Lösungsmittel oder ein Gemisch aus Lösungsmitteln mit einem Siedepunkt unter 400 °C in einer Menge von 0,5 bis 90 Gewichtsprozent, bevorzugt von 5 bis 50 Gewichtsprozent eingesetzt werden. Besonders geeignet sind unverzweigte oder verzweigte Kohlenwasserstoffe wie Pentan, Hexan, Isopentan und zyklische Kohlenwasserstoffe wie Cyclopentan und Cyclohexan.

Als Alkohole können insbesondere die für kosmetische Zwecke üblicherweise verwendeten niederen Alkohole mit 1 bis 4 Kohlenwasserstoffatomen wie zum Beispiel Ethanol und Isopropanol enthalten sein.

Selbstverständlich können die erfindungsgemäßen Mittel auch weitere übliche kosmetische Zusätze wie beispielsweise nichtfestigende nichtionische Polymere, wie zum Beispiel Polyethylenglykol mit einem Molekulargewicht von 600 g/mol, nichtfestigende anionische Polymere und nichtfestigende natürliche Polymere sowie deren Kombination in einer Menge von vorzugsweise 0,01 bis 50 Gewichtsprozent; Parfümöle in einer Menge von vorzugsweise 0,01 bis 5 Gewichtsprozent; Trübungsmittel wie zum Beispiel Ethylenglykoldistearat, in einer Menge von vorzugsweise 0,01 bis 5 Gewichtsprozent; Netzmittel oder Emulgatoren aus den Klassen der anionischen, kationischen, amphoteren oder nichtionogenen oberflächenaktiven Substanzen ohne Waschwirkung wie Fettalkoholsulfate, ethoxylierte Fettalkohole, Fettsäurealkanolamide wie zum Beispiel die Ester der hydrierten Rizinusölfettsäuren in einer Menge von vorzugsweise 0,1 bis 30 Gewichtsprozent; ferner Feuchthaltemittel, Farbstoffe, Lichtschutzmittel, Antioxidantien, Glanzgeber und Konservierungsstoffe in einer Menge von vorzugsweise 0,01 bis 10 Gewichtsprozent enthalten.

Das erfindungsgemäße Mittel kann in verschiedenen Applikationsformen Anwendung finden, wie beispielsweise in Aerosolzubereitungen als Schaum oder als Spray, des weiteren als Non-Aerosol, welches mittels einer Pumpe oder als "Pump and Spray" zum Einsatz kommt. Der Einsatz in üblichen O/W und W/O Emulsionen ist ebenso möglich wie in Anwendungsformen als Gel, Wachs oder Mikroemulsion.

Das erfindungsgemäße Mittel kann auch als färbendes oder pflegendes Haarbehandlungsmittel wie zum Beispiel als Farbfestiger und Haarspülung formuliert sein.

Wenn das erfindungsgemäße Mittel in Form eines Aerosol-Haarsprays oder Aerosol-Haarlackes vorliegt, so enthält es zusätzlich 15 bis 85 Gewichtsprozent, bevorzugt 25 bis 75 Gewichtsprozent, eines Treibmittels und wird in einem Druckbehälter abgefüllt.

Als Treibmittel sind beispielsweise niedere Alkane wie zum Beispiel n-Butan, i-Butan und Propan, oder auch deren Gemische mit Dimethylether sowie ferner bei den in Betracht kommenden Drücken gasförmig vorliegende Treibmittel wie Beispielsweise $N_2$, $N_2O$ und $CO_2$ sowie Gemische der vorstehend genannten Treibmittel geeignet.

Das erfindungsgemäße Mittel zur Festigung der Haare kann auch in Form eines mit Hilfe einer geeigneten mechanisch betriebenen Sprühvorrichtung versprühbaren Non-Aerosol-Haarsprays oder eines Non-Aerosol-Haarlacks vorliegen.

Unter mechanischen Sprühvorrichtungen sind solche Vorrichtungen zu verstehen, welche das Versprühen einer Flüssigkeit ohne Verwendung eines Treibmittels ermöglichen. Als geeignete mechanische Sprühvorrichtung kann beispielsweise eine Sprühpumpe oder ein mit einem Sprühventil versehener elastischer Behälter, in dem das erfindungsgemäße kosmetische Mittel unter Druck abgefüllt wird, wobei sich der elastische Behälter ausdehnt und aus dem das Mittel infolge der Kontraktion des elastischen Behälters bei Öffnen des Sprühventils kontinuierlich abgegeben wird, verwendet werden.

Unter Haarbehandlung soll die Behandlung des menschlichen Kopfhaares vor allem zum Zweck der Herstellung einer Frisur oder zur Pflege der Haare verstanden werden.

Des weiteren ist mit der erfindungsgemäßen Polymerkombination die Herstellung von Konzentraten möglich, welche einen verringerten Wassergehalt oder Lösungsmittelgehalt aufweisen. Die Konzentrate werden nach Transport und gegebenenfalls Lagerung durch Zugabe der erforderlichen Menge Wasser oder Lösungsmittel in ein anwendungsfähiges Haarbehandlungsmittel überführt.

Auswaschversuche in Kombination mit einer meßtechnischen Bestimmung der Wellstabilität (Curl Retention Test) zeigen, daß Haare, die mit dem erfindungsgemäßen Mittel behandelt wurden, auch noch nach dem Auswaschen mit üblichen Shampoos eine spürbare Festigung aufweisen ohne daß eine Schädigung der Haare erfolgt. Erst nach mehreren Haarwäschen ist ein Nachlassen der Festigung der behandelten Haare festzustellen. Die Haare sind nach Behandlung mit dem erfindungsgemäßen Mittel geschmeidig und fühlen sich natürlich an.

Die nachfolgenden Beispiele sollen den Gegenstand der Erfindung näher erläutern:

**Beispiel 1: Flüssigfestiger mit Langzeitwirkung**

| | |
|---|---|
| 6,0 g | Dimethylsiloxan/Methyl-3-mercaptopropylsiloxan/Isobutylmethacrylat Copolymer |
| 50,0 g | Cyclo-octamethyltetrasiloxan |
| 5,0 g | Vinylpyrrolidon/Vinylacetat Copolymer |
| 2,0 g | Dihydroxy-polydimethylsiloxan, 12%ig in Cyclodimethylsiloxan (Abil® OSW 12 der Firma Goldschmidt/ Deutschland) |

(fortgesetzt)

| 37,0 g | Pentan |
| --- | --- |
| 100,0 g | |

## Beispiel 2: Schaumfestiger mit Langzeitwirkung

| 4,0 g | Dimethylsiloxan/Methyl-3-mercaptopropylsiloxan/Isobutylmethacrylat Copolymer |
| --- | --- |
| 20,0 g | Cyclo-octamethyltetrasiloxan |
| 20,0 g | Polydimethylsiloxan (Dow Corning 200 Fluid der Firma Dow Corning/USA) |
| 5,0 g | Dimethiconol, 13%ig in Cyclopolydimethylsiloxan (Dow Corning 1401 Fluid der Firma Dow Corning/USA) |
| 5,0 g | Vinylpyrrolidon/Vinylacetat Copolymer |
| 0,2 g | Cetyltrimethylammoniumchlorid |
| 45.8 g | Isopropanol |
| 100,0 g | |

## Beispiel 3: Sprühfestiger mit Langzeitwirkung

| 3,0 g | Dimethylsiloxan/Methyl-3-mercaptopropylsiloxan/Isobutylmethacrylat Copolymer |
| --- | --- |
| 2,0 g | Dimethiconol in Cyclopolydimethylsiloxan (Dow Corning Fluid 1403 der Firma Dow Corning/USA) |
| 2,0 g | Octylacrylamid/Acrylat/Butylaminoethylmethacrylat Copolymer |
| 0,5 g | Cetyltrimethylammoniumchlorid |
| 92.5 g | Isopropanol |
| 100,0 g | |

## Beispiel 4: Haarcreme zum Festigen mit Langzeitwirkung

| 5,0 g | Dimethylsiloxan/Methyl-3-mercaptopropylsiloxan/Isobutylmethacrylat Copolymer |
| --- | --- |
| 50,0 g | Cyclo-octamethyltetrasiloxan |
| 2,0 g | Acrylat/Alkylacrylat Copolymer (Pemulen TR1 der Firma BF Goodrich Co./USA) |
| 1,0 g | Dihydroxy-polydimethylsiloxan, 13%ig in Cyclodimethylsiloxan (Abil® OSW 13 der Firma Goldschmidt/ Deutschland) |
| 5,0 g | Wasser |
| 37.0 g | Isopropanol |
| 100,0 g | |

## Beispiel 5: Flüssigfarbfestiger mit Langzeitwirkung

| 4,0000 g | Dimethylsiloxan/Methyl-3-mercaptopropylsiloxan/Isobutylmethacrylat Copolymer |
| --- | --- |
| 50,0000 g | Cyclo-octamethyltetrasiloxan |
| 0,5000 g | Dimethiconol (Silicone Fluid F 212 der Firma Wacker/Deutschland) |
| 0,0700 g | 1-Amino-4-(2;3-dihydroxypropyl)amino-5-chlor-2-nitrobenzol |
| 0,0500 g | Basic Brown 17 (C.I. 12251) |
| 0,0023 g | Basic Violett 14 (C.I. 42595) |
| 0,0100 g | Basic Blue 7 (C.I. 42595) |
| 20,0000 g | Pentan |

(fortgesetzt)

| | |
|---|---|
| 25,3677 g | Propanol |
| 100,0000 g | |

**Beispiel 6: Schaumfestiger-Konzentrat mit Langzeitwirkung**

| | |
|---|---|
| 10 g | Dimethylsiloxan/Methyl-3-mercaptopropylsiloxan/Isobutylmethacrylat Copolymer |
| 50 g | Cyclo-octamethyltetrasiloxan |
| 10 g | Dimethiconol (Silicone Fluid F 212 der Firma Wacker/Deutschland) |
| 2 g | Cetyltrimethylammoniumchlorid |
| 6 g | Acrylsäure/Ethylacrylat/N-tert.-Butylacrylamid Copolymer |
| 22 g | Pentan |
| 100 g | |

**Beispiel 7: Haarspray mit Langzeitwirkung**

| | |
|---|---|
| 5,0 g | Dimethylsiloxan/Methyl-3-mercaptopropylsiloxan/Isobutylmethacrylat Copolymer |
| 44,5 g | Cyclo-octamethyltetrasiloxan |
| 0,5 g | Dimethiconol (Silicone Fluid F 212 der Firma Wacker/Deutschland) |
| 5,0 g | Vinylpyrrolidon/Vinylacetat Copolymer |
| 10,0 g | Pentan, |
| 35.0 g | Propan/Butan |
| 100,0 g | |

**Beispiel 8: Flüssigfestiger mit Langzeitwirkung**

| | |
|---|---|
| 6,0 g | Dimethylsiloxan/Methyl-3-mercaptopropylsiloxan/Isobutylmethacrylat |
| 50,0 g | Cyclo-octamethyltetrasiloxan |
| 2,0 g | Dimethicon Copolyol (Abil® B 88183 der Firma Goldschmidt/Deutschland) |
| 5,0 g | Vinylpyrrolidon/Vinylacetat Copolymer |
| 37.0 g | Pentan |
| 100,0 g | |

**Beispiel 9: Schaumfestiger mit Langzeitwirkung**

| | |
|---|---|
| 4,0 g | Dimethylsiloxan/Methyl-3-mercaptopropylsiloxan/Isobutylmethacrylat |
| 20,0 g | Cyclo-octamethyltetrasiloxan |
| 20,0 g | Dimethicone (Abil 10000 der Firma Goldschmidt/Deutschland) |
| 5,0 g | Dimethicone Copolyol (Abil® B 88184 der Firma Goldschmidt/Deutschland) |
| 5,0 g | Vinylpyrrolidon/Vinylacetat/Vinylpropionat Copolymer |
| 0,2 g | Cetyltrimethylammoniumchorid |
| 45,8 g | Isopropanol |
| 100,0 g | |

**Beispiel 10: Sprühfestiger mit Langzeitwirkung**

| | |
|---|---|
| 3,0 g | Dimethylsiloxan/Methyl-3-mercaptopropylsiloxan/Isobutylmethacrylat |
| 2,0 g | Dimethicone (Abil® 10000 der Firma Goldschmidt/Deutschland) |
| 2,0 g | Octylacrylamid/Acrylat/Butylaminoethylmethacrylat Copolymer (Amphomer LV 71 der Firma National Starch/USA) |
| 0,5 g | Cetyltrimethylammoniumchlorid |
| 92.5 g | Isopropanol |
| 100,0 g | |

**Beispiel 11: Haarcreme zum Festigen mit Langzeitwirkung**

| | |
|---|---|
| 5,0 g | Dimethylsiloxan/Methyl-3-mercaptopropylsiloxan/Isobutylmethacrylat |
| 50,0 g | Cyclo-octamethyltetrasiloxan |
| 1,0 g | Dimethicon (Abil® 10000 der Firma Goldschmidt/Deutschland) |
| 2,0 g | Acrylat/C10-30 Alkylacrylat Crosspolymer (Pemulen TR-1 der Firma Goodrich/USA) |
| 5,0 g | Wasser |
| 37.0 g | Isopropanol |
| 100,0 g | |

**Beispiel 12: Flüssigfarbfestiger mit Langzeitwirkung**

| | |
|---|---|
| 4,0 g | Dimethylsiloxan/Methyl-3-mercaptopropylsiloxan/Isobutylmethacrylat |
| 50,0 g | Cyclo-octamethyltetrasiloxan |
| 0,5 g | Dimethicone Copolyol (Abil® B 88184 der Firma Goldschmidt/Deutschland) |
| 0,0700 g | 1-Amino-4-(2,3-dihydroxypropyl)amino-5-chlor-2-nitrobenzol |
| 0,0500 g | Basic Brown 17 (CI 12251) |
| 0,0023 g | Basic Violett 14 (CI 42510) |
| 0,0100 g | Basic Blue 7 (CI 42595) |
| 20,0 g | Pentan |
| ad. | Propanol |
| 100,0 g | |

**Beispiel 13: Schaumfestigerkonzentrat mit Langzeitwirkung**

| | |
|---|---|
| 10,0 g | Dimethylsiloxan/Methyl-3-mercaptopropylsiloxan/Isobutylmethacrylat |
| 50,0 g | Cyclo-octamethyltetrasiloxan |
| 10,0 g | Dimethicone Copolyol (Abil® B 88183 der Firma Goldschmidt/Deutschland) |
| 6,0 g | Acrylat/Acrylamid Copolymer (Ultrahold 8 der Firma BASF/Deutschland) |
| 2,0 g | Cetyltrimethylammoniumchlorid |
| 22,0 g | Pentan |
| 100,0 g | |

Sämtliche angegebenen Prozentzahlen stellen Gewichtsprozent dar, wenn nichts anderes angegeben ist.

**Patentansprüche**

1. Haarbehandlungsmittel mit einem Gehalt an

(a) mindestens einem nichtionischen, wasserunlöslichen Vinyl/Silikon Copolymeren mit einem Grundgerüst aus polymeren Siloxaneinheiten und Seitenketten aus polymeren Vinyleinheiten und
(b) mindestens einem schwerflüchtigen Silikonderivat.

2. Mittel nach Anspruch 1, **dadurch gekennzeichnet, daß** das Vinyl/Silikon Copolymer die allgemeine Formel (I),

$$R^1R^2R^3Si\text{-}[O\text{-}SiR^4R^5\text{-}]_nO\text{-}SiR^6R^7R^8 \qquad (I)$$

aufweist, wobei die Reste $R^1$ bis $R^8$ gleich oder verschieden sein können und unabhängig voneinander für eine Alkyl-, Aryl-, Alkylaryl-, Alkoxy-, Alkylamino-, Fluoroalkyl-, Wasserstoff-, Hydroxyoder für eine ZSA-Gruppe stehen, Z für eine divalente Verbindungsgruppe, S für Schwefel und A für ein aus nichtionischen Monomeren aufgebautes Vinylpolymersegment steht und n eine Zahl größer oder gleich 5 bedeutet mit der Maßgabe, daß mindestens einer der Reste $R^1$ bis $R^8$ für eine ZSA-Gruppe steht.

3. Mittel nach einem der Ansprüche 1 oder 2, **dadurch gekennzeichnet, daß** die nichtionischen Monomeren des Vinylpolymer-Segments A ausgewählt sind aus niedrigpolaren Estern der Acrylsäure oder der Methacrylsäure mit Alkoholen kurzer bis mittlerer Kettenlänge.

4. Mittel nach Anspruch 3, **dadurch gekennzeichnet, daß** das Vinyl/Silikon Copolymer ein Dimethylpolysiloxan/ Methyl-3-mercaptopropylsiloxan/Isobutylmethacrylat Copolymer ist.

5. Mittel nach einem der Ansprüche 1 bis 4, **dadurch gekennzeichnet, daß** das schwerflüchtige Silikonderivat ausgewählt ist aus Polydimethylsiloxanen, Polydimethylsiloxanen mit Hydroxylendgruppen und Dimethylsiloxanglykolcopolymeren.

6. Mittel nach Anspruch 5, **dadurch gekennzeichnet, daß** das schwerflüchtige Silikonderivat ein Polydimethylsiloxan mit Hydroxylendgruppen ist.

7. Mittel nach einem der Ansprüche 1 bis 6, **dadurch gekennzeichnet, daß** das Vinyl/Silikon Copolymer in einer Menge von 0,1 bis 50 Gewichtsprozent und das schwerflüchtige Silikonderivat in einer Menge von 0,01 bis 50 Gewichtsprozent enthalten ist.

8. Mittel nach einem der Ansprüche 1 bis 7, **dadurch gekennzeichnet, daß** es zusätzlich mindestens ein leichtflüchtiges Silikonderivat enthält.

9. Mittel nach Anspruch 8, **dadurch gekennzeichnet, daß** das leichtflüchtige Silikonderivat ausgewählt ist aus leichtflüchtigen linearen oder cyclischen Dimethylpolysiloxanen.

10. Mittel nach einem der Ansprüche 1 bis 9, **dadurch gekennzeichnet, daß** es zusätzlich mindestens ein filmbildendes, haarfestigendes, synthetisches oder natürliches Polymer enthält.

11. Mittel nach einem der Ansprüche 1 bis 10, **dadurch gekennzeichnet, daß** die Komponente (b) eine Verdunstungszahl von größer als 20 aufweist.

12. Verwendung mindestens eines nichtionischen, wasserunlöslichen Vinyl/Silikon Copolymeren mit einem Grundgerüst aus polymeren Siloxaneinheiten und Seitenketten aus polymeren Vinyleinheiten der allgemeinen Formel (I),

$$R^1R^2R^3Si\text{-}[O\text{-}SiR^4R^5\text{-}]_nO\text{-}SiR^6R^7R^8 \qquad (I)$$

wobei die Reste $R^1$ bis $R^8$ gleich oder verschieden sein können und unabhängig voneinander für eine Alkyl-, Aryl-, Alkylaryl-, Alkoxy-, Alkylamino-, Fluoroalkyl-, Wasserstoff-, Hydroxy- oder für eine ZSA-Gruppe stehen, Z für eine divalente Verbindungsgruppe, S für Schwefel und A für ein aus nichtionischen Monomeren aufgebautes Vinylpolymersegment steht und n eine Zahl größer oder gleich 5 bedeutet mit der Maßgabe, daß mindestens einer der Reste $R^1$ bis $R^8$ für eine ZSA-Gruppe steht, zur Erzielung einer langanhaltenden Haarfestigung.

13. Verwendung nach Anspruch 12, **dadurch gekennzeichnet, daß** die nichtionischen Monomeren des Vinylpolymer-Segments A ausgewählt sind aus niedrigpolaren Estern der Acrylsäure oder der Methacrylsäure mit Alkoholen einer Kettenlänge von 1 bis 18 Kohlenstoffatomen.

**Claims**

1. Hair-treatment composition with a content of

   (a) at least one nonionic, water-insoluble vinyl/silicone copolymer with a basic backbone of polymeric siloxane units and side chains of polymeric vinyl units and
   (b) at least one low-volatility silicone derivative.

2. Composition according to Claim 1, **characterized in that** the vinyl/silicone copolymer has the general formula (I)

$$R^1R^2R^3Si\text{-}[O\text{-}SiR^4R^5\text{-}]_nO\text{-}SiR^6R^7R^8 \qquad (I)$$

   where the radicals $R^1$ to $R^8$ may be identical or different and, independently of one another, are an alkyl, aryl, alkylaryl, alkoxy, alkylamino, fluoroalkyl, hydrogen, hydroxyl or a ZSA group, Z is a divalent linking group, S is sulphur and A is a vinyl polymer segment constructed from nonionic monomers, and n is a number greater than or equal to 5, with the proviso that at least one of the radicals $R^1$ to $R^8$ is a ZSA group.

3. Composition according to one of Claims 1 and 2, **characterized in that** the nonionic monomers of the vinyl polymer segment A are chosen from low-polarity esters of acrylic acid or of methacrylic acid with alcohols of short to medium chain length.

4. Composition according to Claim 3, **characterized in that** the vinyl/silicone copolymer is a dimethylpolysiloxane/ methyl-3-mercaptopropylsiloxane/isobutyl methacrylate copolymer.

5. Composition according to one of Claims 1 to 4, **characterized in that** the low-volatility silicone derivative is chosen from polydimethylsiloxanes, polydimethylsiloxanes with hydroxyl end groups and dimethylsiloxane glycol copolymers.

6. Composition according to Claim 5, **characterized in that** the low-volatility silicone derivative is a polydimethylsiloxane with hydroxyl end groups.

7. Composition according to one of Claims 1 to 6, **characterized in that** the vinyl/silicone copolymer is present in an amount of from 0.1 to 50% by weight and the low-volatility silicone derivative is present in an amount of from 0.01 to 50% by weight.

8. Composition according to one of Claims 1 to 7, **characterized in that** it additionally comprises at least one readily volatile silicone derivative.

9. Composition according to Claim 8, **characterized in that** the readily volatile silicone derivative is chosen from readily volatile linear or cyclic dimethylpolysiloxanes.

10. Composition according to one of Claims 1 to 9, **characterized in that** it additionally comprises at least one film-forming, hair-setting, synthetic or natural polymer.

11. Composition according to one of Claims 1 to 10, **characterized in that** component (b) has an evaporation number greater than 20.

**12.** Use of at least one nonionic, water-insoluble vinyl/silicone copolymer with a basic backbone of polymeric siloxane units and side chains of polymeric vinyl units of the general formula (I),

$$R^1R^2R^3Si\text{-}[O\text{-}SiR^4R^5\text{-}]_nO\text{-}SiR^6R^7R^8 \qquad \text{(I)}$$

where the radicals $R^1$ 1 to $R^8$ may be identical or different and, independently of one another, are an alkyl, aryl, alkylaryl, alkoxy, alkylamino, fluoroalkyl, hydrogen, hydroxyl or a ZSA group, Z is a divalent linking group, S is sulphur and A is a vinyl polymer segment constructed from nonionic monomers, and n is a number greater than or equal to 5, with the proviso that at least one of the radicals $R^1$ to $R^8$ is a ZSA group, for achieving a long-lasting setting of hair.

**13.** Use according to Claim 12, **characterized in that** the nonionic monomers of the vinyl polymer segment A are chosen from low-polarity esters of acrylic acid or of methacrylic acid with alcohols having a chain length of from 1 to 18 carbon atoms.

**Revendications**

**1.** Composition de traitement capillaire comprenant

(a) au moins un copolymère vinyle/silicone non ionique insoluble dans l'eau, présentant un squelette composé de motifs siloxane polymères et des chaînes latérales composées de motifs vinyle polymères et
(b) au moins un dérivé siliconé peu volatil.

**2.** Composition selon la revendication 1, **caractérisée en ce que** le copolymère vinyle/silicone est de formule générale (I),

$$R^1R^2R^3Si\text{-}[O\text{-}SiR^4R^5\text{-}]_nO\text{-}SiR^6R^7R^8 \qquad \text{(I)}$$

dans laquelle les radicaux $R^1$ à $R^8$ peuvent être identiques ou différents et représentent indépendamment les uns des autres, un groupe alkyle, aryle, alkylaryle, alcoxy, alkylamino, fluoroalkyle, hydrogène, hydroxy ou ZSA, Z représente un groupe de liaison bivalent, S représente le soufre et A représente un segment de polymère vinylique composé de monomères non ioniques, et n représente un nombre supérieur ou égal à 5, à condition qu'au moins l'un des radicaux $R^1$ à $R^8$ représente un groupe ZSA.

**3.** Composition selon l'une quelconque des revendications 1 ou 2, **caractérisée en ce que** les monomères non ioniques du segment de polymère vinylique A sont choisis parmi des esters peu polaires de l'acide acrylique ou de l'acide méthacrylique avec des alcools de longueur de chaîne courte à moyenne.

**4.** Composition selon la revendication 3, **caractérisée en ce que** le copolymère vinyle/silicone est un copolymère diméthylpolysiloxane/méthyl-3-mercaptopropylsiloxane/méthacrylate d'isobutyle.

**5.** Composition selon l'une quelconque des revendications 1 à 4, **caractérisée en ce que** le dérivé siliconé peu volatil est choisi parmi des polydiméthylsiloxanes, des polydiméthylsiloxanes ayant des groupes terminaux hydroxy et des copolymères diméthylsiloxane-glycol.

**6.** Composition selon la revendication 5, **caractérisée en ce que** le dérivé siliconé peu volatil est un polydiméthylsiloxane ayant des groupes terminaux hydroxy.

**7.** Composition selon l'une quelconque des revendications 1 à 6, **caractérisée en ce que** le copolymère vinyle/silicone est présent en une quantité de 0,1 à 50 pour cent en poids et le dérivé siliconé peu volatil est présent en une quantité de 0,01 à 50 pour cent en poids.

**8.** Composition selon l'une quelconque des revendications 1 à 7, **caractérisée en ce qu'**elle comprend en outre au moins un dérivé siliconé volatil.

**9.** Composition selon la revendication 8, **caractérisée en ce que** le dérivé siliconé volatil est choisi parmi des diméthylpolysiloxanes linéaires ou cycliques volatils.

**10.** Composition selon l'une quelconque des revendications 1 à 9, **caractérisée en ce qu'**elle comprend en outre au moins un polymère filmogène fixateur de cheveux, synthétique ou naturel..

**11.** Composition selon l'une quelconque des revendications 1 à 10, **caractérisée en ce que** le composant (b) présente un indice d'évaporation supérieur à 20.

**12.** Utilisation d'au moins un copolymère vinyle/silicone non ionique insoluble dans l'eau, présentant un squelette composé de motifs siloxane polymères et des chaînes latérales composées de motifs vinyle polymères de formule générale (I),

$$R^1R^2R^3Si\text{-}[O\text{-}SiR^4R^5\text{-}]_nO\text{-}SiR^6R^7R^8 \qquad (I)$$

dans laquelle les radicaux $R^1$ à $R^8$ peuvent être identiques ou différents et représentent indépendamment les uns des autres, un groupe alkyle, aryle, alkylaryle, alcoxy, alkylamino, fluoroalkyle, hydrogène, hydroxy ou ZSA, Z représente un groupe de liaison bivalent, S représente le soufre et A représente un segment de polymère vinylique composé de monomères non ioniques, et n représente un nombre supérieur ou égal à 5, à condition qu'au moins l'un des radicaux $R^1$ à $R^8$ représente un groupe ZSA, en vue d'obtenir une fixation durable des cheveux.

**13.** Utilisation selon la revendication 12, **caractérisée en ce que** les monomères non ioniques du segment de polymère vinylique A sont choisis parmi des esters peu polaires de l'acide acrylique ou de l'acide méthacrylique avec des alcools ayant une longueur de chaîne de 1 à 18 atomes de carbone.